# FASCICULE DE BREVET EUROPEEN

(11) **EP 1 196 638 B1**
(45) Date de publication et mention de la délivrance du brevet: **29.01.2014**
(21) Numéro de dépôt: 00948072.4
(22) Date de dépôt: 03.07.2000
(51) Int. Cl.: C12Q 1/68, A61P 43/00

(54) **UTILISATION DU GENE KRIT1 DANS LE DOMAINE DE L'ANGIOGENESE**
VERWENDUNG DES KRIT1 GENS IM ZUSAMMENHANG MIT ANGIOGENESE
USE OF THE KRIT1 GENE IN ANGIOGENESIS

(30) Priorité: 01.07.1999 FR 9908504
(43) Date de publication de la demande: 17.04.2002
(73) Titulaire: INSTITUT NATIONAL DE LA SANTE ET DE LA RECHERCHE MEDICALE (INSERM), 75013 Paris (FR)
(72) Inventeur: TOURNIER-LASSERVE Elisabeth, F-75016 Paris (FR); LABERGE-LE-COUTEULX, Sophie, F-76000 Rouen (FR); LABAUGE, Pierre, F-34090 Montpellier (FR)
(74) Mandataire: Regimbeau
(86) Numéro de dépôt international: PCT/FR2000/001887
(87) Numéro de publication internationale: WO 2001/002604

(56) Documents cités:
- LABERGE ET AL.: "FAMILIAL CAVERNOUS ANGIOMAS CCM1 GENE" EUR.J.HUM.GEN., vol. 6/suppl.1, mai 1998 (1998-05), page 146/P4.168 XP002136142
- SEREBRIISKII ET AL.: "ASSOCIATION OF KREV-1/RAP1A WITH KRIT1, A NOVEL ANKYRIN REPEAT-CONTAINING PROTEIN ENCODED BY A GENE MAPPING TO 7q21-22" ONCOGENE, vol. 15, 1997, pages 1043-1049, XP000892143
- LABERGE ET AL.: "GENETIC HETEROGENEITY AND ABSENCE OF FOUNDER EFFECT IN A SERIES OF 36 FRENCH CEREBRAL CAVERNOUS ANGIOMAS FAMILIES" EUR.J.HUM.GEN., vol. 7, mai 1999 (1999-05), pages 499-504, XP000892146
- CRAIG ET AL.: "MULTILOCUS LINKAGE IDENTIFIES TWO NEW LOCI FOR A MENDELIAN FORM OF STROKE, CEREBRAL CAVERNOUS MALFORMATION, AT 7p15-13 AND 3q25.2-27" HUM.MOL.GEN., vol. 7, no. 12, 1998, pages 1851-1858, XP002136143
- SAHOO ET AL.: "Mutations in the gene encoding KRIT1, a Krev-1/rap1a binding protein, cause cerebral cavernous malformations (CCM1) " HUM.MOL.GEN., vol. 8, no. 12, novembre 1999 (1999-11), XP002136144
- COUTEULX SOPHIE LABERGE-LE ET AL: "Truncating mutations in CCM1, encoding KRIT1, cause hereditary cavernous angiomas." NATURE GENETICS, vol. 23, no. 2, octobre 1999 (1999-10), pages 189-193, XP002151276 ISSN: 1061-4036

### LISTAGE DE SEQUENCE

<110> INSTITUT NATIONAL DE LA SANTE ET DE LA RECHERCHE MEDICALE - INSERM
<120> D18210
<130> Utilisation du gène *Krit1* dans le domaine de l'angiogenèse
<140>
   <141>
<160> 51
<170> PatentIn Vers. 2.0
<210> 1
   <211> 18
   <212> ADN
   <213> Artificial Sequence
<220>
   <223> Amorce sens
<400> 1
   gagcggataa aaactaat 18
<210> 2
   <211> 18
   <212> ADN
   <213> Artificial Sequence
<220>
   <223> Amorce reverse
<400> 2
   gagctaaaat tcattcaa 18
<210> 3
   <211> 18
   <212> ADN
   <213> Artificial Sequence
<220>
   <223> Amorce sens
<400> 3
   gctcttaatg ggtttttg 18
<210> 4
   <211> 18
   <212> ADN
   <213> Artificial Sequence
<220>
   <223> Amorce reverse
<400> 4
   agcaatgtgg agtaaaac 18
<210> 5
   <211> 18
   <212> ADN
   <213> Artificial Sequence
<220>
   <223> Amorce sens
<400> 5
   tttggaatga gaacagtc 18
<210> 6
   <211> 18
   <212> ADN
   <213> Artificial Sequence
<220>
   <223> Amorce reverse
<400> 6
   gtcctgttgt atttttca 18
<210> 7
   <211> 18
   <212> ADN
   <213> Artificial Sequence
<220>
   <223> Amorce sens
<400> 7
   gttgttgttt tttgtttg 18
<210> 8
   <211> 18
   <212> ADN
   <213> Artificial Sequence
<220>
   <223> Amorce reverse
<400> 8
   acctggaaaa taacttac 18
<210> 9
   <211> 18
   <212> ADN
   <213> Artificial Sequence
<220>
   <223> Amorce sens
<400> 9
   atgtaatgcc ttttttcc 18
<210> 10
   <211> 18
   <212> ADN
   <213> Artificial Sequence
<220>
   <223> Amorce reverse
<400> 10
   atgcctggct ctaactat 18
<210> 11
   <211> 18
   <212> ADN
   <213> Artificial Sequence
<220>
   <223> Amorce sens
<400> 11
   ttgttagatt gtgatgta 18
<210> 12
   <211> 18
   <212> ADN
   <213> Artificial Sequence
<220>
   <223> Amorce reverse
<400> 12
   aacataataa aaactttc 18
<210> 13
   <211> 18
   <212> ADN
   <213> Artificial Sequence
<220>
   <223> Amorce sens
<400> 13
   tttataaaag gaatgatg 18
<210> 14
   <211> 18
   <212> ADN
   <213> Artificial Sequence
<220>
   <223> Amorce reverse
<400> 14
   tcaactcaaa ccatatca 18
<210> 15
   <211> 18
   <212> ADN
   <213> Artificial Sequence
<220>
   <223> Amorce sens
<400> 15
   tgtagcctaa taaccaaa 18
<210> 16
   <211> 18
   <212> ADN
   <213> Artificial Sequence
<220>
   <223> Amorce reverse
<400> 16
   agcatagcac aagaccat 18
<210> 17
   <211> 18
   <212> ADN
   <213> Artificial Sequence
<220>
   <223> Amorce sens
<400> 17
   ggtgaagttt ttaatatg 18
<210> 18
   <211> 18
   <212> ADN
   <213> Artificial Sequence
<220>
   <223> Amorce reverse
<400> 18
   caatagttta tgaagtcc 18
<210> 19
   <211> 18
   <212> ADN
   <213> Artificial Sequence
<220>
   <223> Amorce sens
<400> 19
   atatttacaa aggcaagc 18
<210> 20
   <211> 18
   <212> ADN
   <213> Artificial Sequence
<220>
   <223> Amorce reverse
<400> 20
   tgacatgatt ggtaaaaa 18
<210> 21
   <211> 18
   <212> ADN
   <213> Artificial Sequence
<220>
   <223> Amorce sens
<400> 21
   tggtacattt tcctttca 18
<210> 22
   <211> 18
   <212> ADN
   <213> Artificial Sequence
<220>
   <223> Amorce reverse
<400> 22
   ctttatgatt gctggggc 18
<210> 23
   <211> 18
   <212> ADN
   <213> Artificial Sequence
<220>
   <223> Amorce sens
<400> 23
   ggtgaagttt ttaatatg 18
<210> 24
   <211> 18
   <212> ADN
   <213> Artificial Sequence
<220>
   <223> Amorce reverse
<400> 24
   caatagttta tgaagtcc 18
<210> 25
   <211> 18
   <212> ADN
   <213> Artificial Sequence
<220>
   <223> Amorce sens
<400> 25
   aatagatagg gaactgcc 18
<210> 26
   <211> 18
   <212> ADN
   <213> Artificial Sequence
<220>
   <223> Amorce reverse
<400> 26
   gtggcttgag taacagtt 18
<210> 27
   <211> 18
   <212> ADN
   <213> Artificial Sequence
<220>
   <223> Amorce sens
<400> 27
   taatgcccac tgaaagaa 18
<210> 28
   <211> 18
   <212> ADN
   <213> Artificial Sequence
<220>
   <223> Amorce reverse
<400> 28
   ggctggtctt gaactctg 18
<210> 29
   <211> 20
   <212> ADN
   <213> Homo sapiens
<400> 29
   atcaggtcag acagaaaact 20
<210> 30
   <211> 20
   <212> ADN
   <213> Homo sapiens
<400> 30
   tacaaatcgg gtaagagttg 20
<210> 31
   <211> 20
   <212> ADN
   <213> Homo sapiens
<400> 31
   ccctttctag gtagataaag 20
<210> 32
   <211> 20
   <212> ADN
   <213> Homo sapiens
<400> 32
   cagaagacaa gtactgtttc 20
<210> 33
   <211> 20
   <212> ADN
   <213> Homo sapiens
<400> 33
   taatgattag ggaacgacag 20
<210> 34
   <211> 20
   <212> ADN
   <213> Homo sapiens
<400> 34
   atgcatgctg gtaaatggaa 20
<210> 35
   <211> 20
   <212> ADN
   <213> Homo sapiens
<400> 35
   ttttatacag gtatggaaaa 20
<210> 36
   <211> 20
   <212> ADN
   <213> Homo sapiens
<400> 36
   aacggataga gtaagttatt 20
<210> 37
   <211> 20
   <212> ADN
   <213> Homo sapiens
<400> 37
   acatttctag catataacag 20
<210> 38
   <211> 20
   <212> ADN
   <213> Homo sapiens
<400> 38
   taacaaacca gtaagaatta 20
<210> 39
   <211> 20
   <212> ADN
   <213> Homo sapiens
<400> 39
   tttcttgtag tatgaaaaag 20
<210> 40
   <211> 20
   <212> ADN
   <213> Homo sapiens
<400> 40
   gaaaacctca gtaagaaagt 20
<210> 41
   <211> 20
   <212> ADN
   <213> Homo sapiens
<400> 41
   tgtttttcag gccttcaact 20
<210> 42
   <211> 20
   <212> ADN
   <213> Homo sapiens
<400> 42
   tgaaaaacag gtttgcttgg 20
<210> 43
   <211> 20
   <212> ADN
   <213> Homo sapiens
<400> 43
   ttcctttaag attgaagacc 20
<210> 44
   <211> 20
   <212> ADN
   <213> Homo sapiens
<400> 44
   gtttcctaaa gtaagtattt 20
<210> 45
   <211> 20
   <212> ADN
   <213> Homo sapiens
<400> 45
   gtgcttacag tgaagaaaat 20
<210> 46
   <211> 20
   <212> ADN
   <213> Homo sapiens
<400> 46
   tgaatacaag gtaagctgtt 20
<210> 47
   <211> 20
   <212> ADN
   <213> Homo sapiens
<400> 47
   ttgtttttag aatctcagta 20
<210> 48
   <211> 20
   <212> ADN
   <213> Homo sapiens
<400> 48
   ggaaactaag gtagattttc 20
<210> 49
   <211> 20
   <212> ADN
   <213> Homo sapiens
<400> 49
   tatgttgcag gctttactca 20
<210> 50
   <211> 20
   <212> ADN
   <213> Homo sapiens
<400> 50
   tacaaaacag gtaagtatca 20
<210> 51
   <211> 20
   <212> ADN
   <213> Homo sapiens
<400> 51
   tactttgtag gctctggtcg 20

## Description

La présente invention a pour objet une méthode de diagnostic des angiomes caverneux ou cavernomes pour la détection de mutations dans le gène *Krit1.* En particulier, cette détection est mise en oeuvre au moyen de séquences nucléotidiques telles que définies dans la revendication 8.

Les cavernomes sont des malformations vasculaires le plus souvent localisées dans le système nerveux central mais également dans la rétine, le foie, les reins, etc.. et sont caractérisés par des cavités capillaires anormalement élargies sans intervention du parenchyme cérébral (Russell et al.). Les symptômes cliniques comprennent des céphalées, des hémorragies, des crises d'épilepsie et des déficits neurologiques focaux. La prévalence des angiomes caverneux est proche de 0,5 % dans la population générale (Otten et al.). Ces angiomes peuvent être transmis de façon héréditaire sous une forme autosomique dominante dans près de 50 % des cas (Rigamonti et al.). 3 localisations (ou loci) de malformations caverneuses cérébrales (CCM) ont été identifiées sur le bras long du chromosome 7, le bras court du chromosome 7 et le bras long du chromosome 3 (7q, 7p et 3q respectivement). Un important effet fondateur a été observé dans la population hispano-américaine, dans laquelle toutes les familles étaient liées au locus *CCM1* localisé sur 7q (Rigamonti et al. ; Dubovsky et al. ; Günel et al. ; et Craig et al.).

Laberge et al, 1998, (Eur. J. Hum. Gen., vol. 6 / suppl. 1, p. 146, P4.167) enseigne que le locus CCM1, comprenant les gènes CDK1, PAS1, HUMLD14 et KRIT1, est associé à la présence de cavernomes.

Les inventeurs ont récemment établi les caractéristiques génétiques et cliniques des cavemomes, ainsi que les caractéristiques héréditaires dans une série de 57 familles françaises (Labauge et al.). Des investigations de neuro-imagerie ont confirmé la grande fréquence de lésions multiples dans les cavernomes héréditaires. Une corrélation hautement significative a également été mise en évidence entre le nombre de lésions et l'âge du patient, ce qui suggère fortement la nature dynamique de ces malformations vasculaires appelées également hamartomes. L'analyse de liaison génétique conduite dans 36 de ces familles a montré que 65 % d'entre elles étaient liées au locus *CCM1* avec aucun effet fondateur (Laberge et al.).

La taille de l'intervalle génétique contenant le locus *CCM1* avait été réduite en 1995 à 4 centimorgans, le locus *CCM1* étant encadré par D7S2410 et D7S689 (Johnson et al.). Au moyen essentiellement d'une approche *in silico*, les inventeurs ont établi une carte physique et transcriptionnelle de l'intervalle de *CCM1.* Parmi les 53 unités transcriptionnelles cartographiées à l'intérieur de la région critique, l'une d'elles correspondait à *Krit1*, un gène dont le produit interagit avec Rap1A (également appelé Krev1), un membre de la famille des gènes Ras impliqués dans la prolifération cellulaire, la différentiation et la morphogenèse (Bos et al.). En utilisant en combinaison la technique SSCP et le séquençage, les inventeurs ont identifié dans 8 familles *CCM1* non apparentées des mutations conduisant très probablement à une protéine Krit1 tronquée. La coségrégation de ces mutations avec le phénotype affecté suggère fortement que *Krit1* est la protéine mutée dans les familles atteintes de cavernomes liés au locus *CCM1* et suggère l'implication de la voie de transduction du signal de Rap1A dans la vasculogénèse et/ou l'angiogénèse.

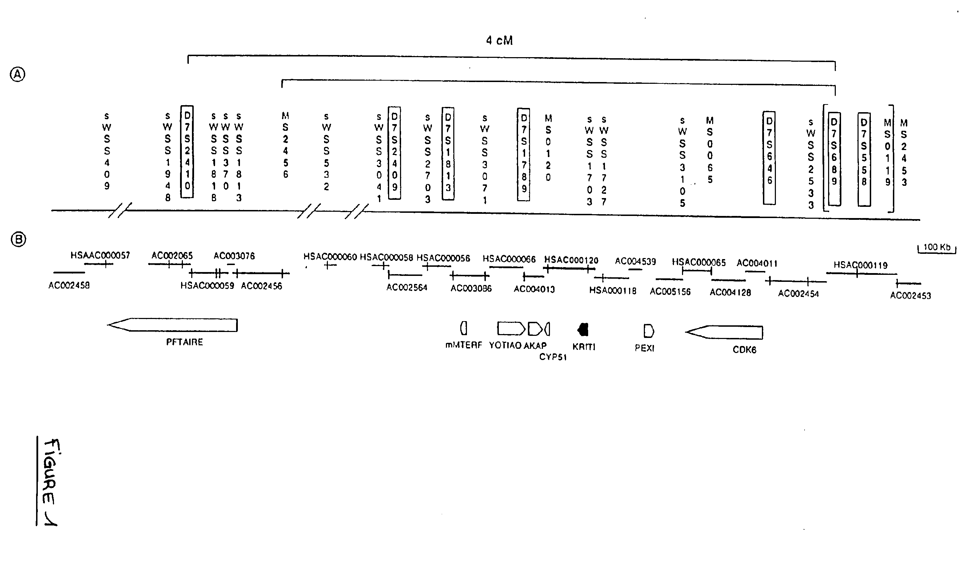

En utilisant un contig de YAC préalablement publié, et les bases de données de séquences publiques (The Washington University Chromosome 7 Project), les inventeurs ont construit des contigs de BAC/PAC couvrant 90 % de l'intervalle *CCM1,* estimé à 1 600 Kb (Figure 1). 20 familles comprenant 179 méioses potentiellement informatives et dont il avait été précédemment montré qu'elles avaient une probabilité a posteriori d'être liées au locus *CCM1* supérieure à 90 % ont été utilisées pour cartographier finement ce locus avec des marqueurs polymorphes identifiés au moyen des séquences BAC/PAC (Figure 1). Un événement de recombinaison observé chez un individu affecté (famille 27 dans Labauge et al.) a permis aux inventeurs de réduire légèrement cet intervalle qui est maintenant encadré par M2456 (limite centromérique) et D7S689 (limite télomérique). Le criblage de banques de données publiques telles que Gene Map du Human Genome, Unigene et dBEST a permis aux inventeurs de cartographier à l'intérieur de cet intervalle 574 *Expressed Sequence Tags* (EST) qui ont ensuite été regroupées en 53 unités transcriptionnelles putatives comprenant 6 gènes déjà connus : CDK6, HUMI.D14, KRIT1, PEX-1, mMTERF et Yotiao.

*Krit1* avait été identifié lors d'un criblage destiné à identifier les protéines interagissant avec Rap1A/Krev1, un membre de la famille des gènes Ras (Serebriiski et al.). Il code pour une protéine de 529 acides aminés qui comprend 4 domaines ankyrines et interagit avec Rap1A/Krev1 au moyen de sa région carboxyterminale. Il a déjà été indiqué que l'ARN messager de *Krit1* était exprimé à des niveaux faibles dans de nombreux tissus y compris le cerveau. Bien que la fonction exacte de *Krit1* soit encore inconnue, les inventeurs ont considéré qu'il était un bon gène candidat pour *CCM1,* et ceci pour plusieurs raisons. Rap1A / Krev1A a été identifié sur la base de son homologie avec *Dras3,* un homologue de Ras chez la drosophile, ainsi que sur la base de son activité anti-mitogène dans des fibroblastes transformés par K-ras (Pizon et al., 1988 / Kitayama et al., 1989). Bien que la pertinence physiologique de cet effet anti-mitogène observé *in vitro* n'ait pas encore été établie *in vivo,* ceci a conduit à considérer que cette protéine était un antagoniste de Ras. Un rôle de la voie de signalisation de Ras dans la vasculogenèse et l'angiogenèse a été fortement suggéré par les anomalies vasculaires observées dans les modèles murins invalidés pour les protéines impliquées dans cette voie, par exemple les protéines raf ou GAP120 (Henkemeyer et al., 1995 ; Wojnowski et al., 1997). En plus de ce rôle putatif en tant qu'antagoniste de Ras, Rap1A / Krev1 a été impliqué dans la différentiation cellulaire et la morphogenèse (Asha et al., 1999 ; Quarck et al., 1996 ; Pizon et al., 1988).

En d'autres termes, dans la mesure où la protéine Krit1 tronquée donne lieu à une anomalie de l'angiogenèse s'accompagnant d'une prolifération des cellules endothéliales, il est raisonnable d'en déduire que le gène *Krit1* pourrait avoir un rôle de contrôle de l'angiogenèse.

Ainsi, dans le cadre de la présente invention, les inventeurs ont montré que des mutations dans le gène *Krit1* susceptibles de donner lieu à une protéine Kritl tronquée sont responsables de l'apparition d'anomalies vasculaires. Ces anomalies vasculaires peuvent affecter différents territoires incluant le cerveau et la peau et prendre différentes formes (cavemomes, angiomes capillaro-veineux). Le type des lésions observées (développement de maladies vasculaires anormales combiné à la nature des mutations observées (mutations entraînant une troncation de la protéine) suggèrent fortement que cette protéine exerce un contrôle de l'angiogenèse qui pourrait être susceptible d'applications thérapeutique dans le domaine de l'antiangiogenèse, en particulier dans le domaine tumoral.

L'alignement de l'ADNc de *Krit1* avec le BAC AC000020, l'un des BAC localisé dans l'intervalle, a permis aux inventeurs de déterminer la structure génomique de *Krit1.* Ce gène est codé par 12 exons qui sont tous compris dans le BAC AC000020. Les inventeurs ont dessiné les amorces oligonucléotidiques introniques destinées à amplifier les exons (Tableau n° 1) ainsi que les séquences de jonction (Tableau n° 2). Ces amorces ont été particulièrement délicates à mettre au point en raison de la richesse en bases A et T de *Krit1* et sont très spécifiques de *Krit1.* Ainsi, les amorces de séquence nucléotidique choisie dans le groupe comprenant SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ N° 27, SEQ ID N° 28 sont ici décrites.

Au moyen de ces amorces, les inventeurs ont pu amplifier tous les exons. Un ensemble de 20 patients CCM non apparentés appartenant à des familles dans lesquelles l'analyse HOMOG a montré une probabilité *a posteriori* d'être liée au locus *CCM1* supérieure à 90 % a permis de procéder à des criblages de mutations par une analyse combinant une approche de type Single Strand Conformation Polymorphism (SSCP), dans 4 conditions distinctes, et une approche de séquençage.

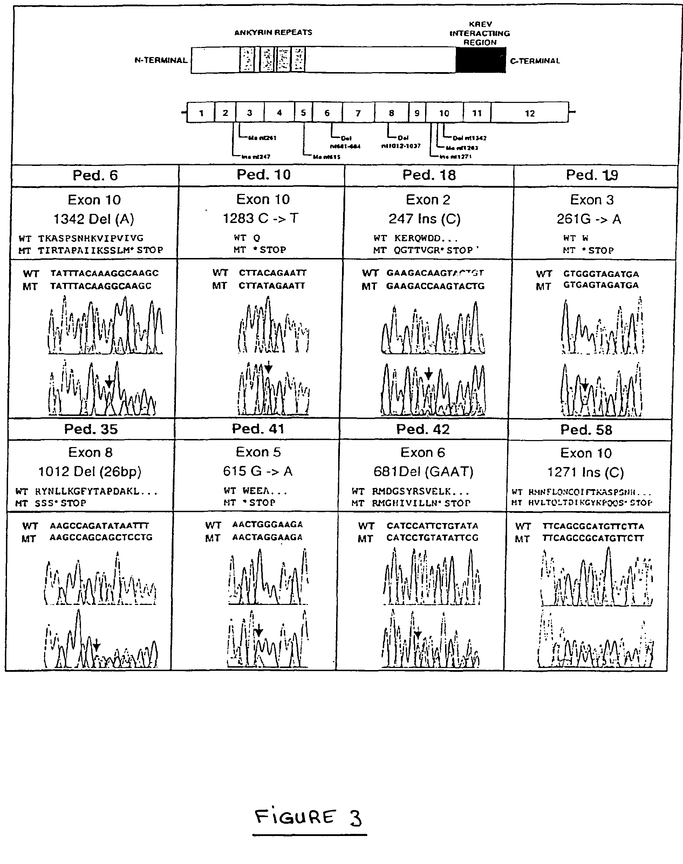

Les produits amplifiés de 8 de ces patients ont montré des variants de conformation anormaux qui n'ont été observés dans aucun des 50 sujets contrôles. L'analyse de la séquence de ces amplimères a révélé des mutations hétérozygotes dans ces 8 patients (Tableau 3 et Figure 3). Ces mutations coségrégeaient avec le phénotype malade dans les 8 familles de ces patients.

La présente invention a également pour objet l'utilisation d'au moins une séquence nucléotidique telle que définie ci-dessus pour la détection, à partir d'un prélèvement biologique, de la présence d'une mutation dans le gène *Krit1* susceptible de conduire à une protéine krit1 tronquée; de préférence une mutation liée à la survenue d'anomalies vasculaires telles que définies précédemment. De manière préférentielle, le prélèvement biologique est du sang.

Plus particulièrement, le pedigree 6 présente une délétion d'un A au nucléotide 1342, dans l'exon 10. Cette délétion conduit à un changement du cadre de lecture et ainsi à un codon stop prématuré. Dans le pedigree 10, une substitution de C par T au nucléotide 1283 dans l'exon 10 conduit au remplacement d'une glutamine par un codon stop. Le pedigree 58 montre quant à lui l'insertion d'un C après le nucléotide 1271, également dans l'exon 10, ce qui entraîne un changement du cadre de lecture et un codon stop prématuré. Le pedigree 41 montre une substitution de G par A au nucléotide 615 ce qui conduit au remplacement d'un tryptophane par un codon stop prématuré dans l'exon 5. Le pedigree 42 présente une délétion de 4 pb (nucléotides 681-684) dans l'exon 6 donnant lieu à un codon stop prématuré. Le pedigree 35 présente une délétion de 26 pb (nucléotides 1012-1037) à l'intérieur de l'exon 8 ; cette délétion causant un changement du cadre de lecture et un codon stop prématuré au codon 332. Le pedigree 18 présente une insertion d'un C à l'intérieur de l'exon 2 après le nucléotide 247 ; cette insertion conduit à un changement du cadre de lecture et à un codon stop prématuré. Le pedigree 19 montre une substitution d'un G par un A au nucléotide 261 ; celle-ci entraîne un changement de cadre de lecture ainsi qu'un codon stop prématuré au codon 79.

Les analyses SSCP des membres affectés et non affectés ont montré la coségrégation parfaite des mutations avec le phénotype affecté dans chacun de ces 8 pedigrees (Figure 3).

Ainsi, les séquences nucléotidiques conformes à la présente invention sont utilisées pour procéder à la détection d'une mutation dans au moins un exon du gène *Krit1* susceptible de conduire à une protéine Krit1 tronquée. Plus particulièrement, ces séquences nucléotidiques peuvent être utilisées par couple compte tenu de leur spécificité d'un exon selon la répartition suivante :
- SEQ ID N° 1 / SEQ ID N° 2 pour l'exon 1,
- SEQ ID N° 3 / SEQ ID N° 4 pour l'exon 2,
- SEQ ID N° 5 / SEQ ID N° 6 pour l'exon 3,
- SEQ ID N° 7 / SEQ ID N° 8 pour l'exon 4,
- SEQ ID N° 9 / SEQ ID N° 10 pour l'exon 5,
- SEQ ID N° 11 / SEQ ID N° 12 pour l'exon 6,
- SEQ ID N° 13 / SEQ ID N° 14 pour l'exon 7,
- SEQ ID N° 15 / SEQ ID N° 16 pour l'exon 8,
- SEQ ID N° 17 / SEQ ID N° 18 pour l'exon 9,
- SEQ ID N° 19 / SEQ ID N° 20 pour l'exon 10,
- SEQ ID N° 21 / SEQ ID N° 22 pour l'exon 10,
- SEQ ID N° 23 / SEQ ID N° 24 pour l'exon 11,
- SEQ ID N° 25 / SEQ ID N° 26 pour l'exon 12,
- SEQ ID N° 27 / SEQ ID N° 28 pour l'exon 12.

Avantageusement, la détection d'une mutation dans Krit1 est précédée de l'amplification de l'exon dans lequel la mutation est recherchée et cette amplification peut être réalisée par PCR ou PCR-like.

La nature tronquante de ces mutations, leur absence chez les contrôles sains et leur conségrégation avec le phénotype affecté suggère fortement qu'il s'agit de mutations délétères dans ces familles.

Les inventeurs n'ont pas détecté de variants de conformation anormaux de SSCP dans 12 des 20 familles testées. Plusieurs hypothèses peuvent être émises pour expliquer ceci. La sensibilité de SSCP n'est pas de 100 % même lorsqu'on utilise plusieurs types de conditions comme cela a été le cas ici. De façon intéressante, aucun de ces variants de conformation anormaux n'a été observé dans le premier criblage qui a été réalisé à 20°C sans glycérol. De plus, les délétions qui emporteraient les régions contenant les séquences hybridant avec les amorces n'auraient pu être détectées par cette approche. Enfin, certaines de ces familles, bien que montrant une forte probabilité d'être liées au locus *CCM1,* pourraient en fait être liées à l'un des autres loci *CCM.*

La présente invention a également pour objet une méthode de diagnostic génotypique de cavernomes chez un individu, caractérisée par le fait que l'on prélève un échantillon biologique dudit individu, que l'on détecte la présence d'une mutation dans le gène *Krit1* conduisant à l'expression d'une protéine Krit1 tronquée par analyse de la séquence nucléique présente dans ledit échantillon, une telle mutation étant liée à la survenue de cavernomes.

La séquence d'acides nucléiques analysée peut être indifféremment de l'ADN génomique, de l'ADNc ou de l'ARNm. L'analyse peut être réalisée par hybridation, par séquençage ou par migration électrophorétique, en particulier par SSCP ou DGGE (Electrophorèse en gel en gradient dénaturant). La détection de ces mutations pourrait également être réalisée à l'aide d'une méthodologie permettant de détecter directement la présence de la protéine tronquée, par exemple les méthodes dites de Protein Truncation Test (traduction in vitro de transcrits reverse d'ADNc suivie d'une révélation de la protéine par anticorps ou après marquage de la protéine à l'aide d'un acide aminé marqué). Enfin, la recherche de mutations peut être faite par analyse directe du transcrit reverse d'ADNc préparé à partir des ARN totaux (en particulier provenant de cellules transformées par le virus EBV, cellules dans lesquelles les auteurs ont montré l'expression du transcrit de Krit1).

Avantageusement, tout ou partie de la séquence d'acides nucléiques correspondant au gène *Krit1* est amplifiée préalablement à la détection de la présence d'une mutation, cette amplification pouvant être réalisée par PCR ou PCR-like. De façon tout à fait préférentielle, cette amplification peut être réalisée au moyen d'amorces choisies parmi les séquences d'acides nucléiques conformes à la présente invention, par exemple utilisées selon la répartition ci-dessus mentionnée.

La question principale est donc de comprendre comment ces mutations ont pu conduire aux cavernomes. Peu de choses sont connues en réalité sur la nature de ces lésions qui sont considérées comme des malformations vasculaires ou hamartomes. Il semblerait que la période d'apparition de ces malformations pendant la vie embryonnaire n'est pas tout à fait claire. De plus, dans certains cas, particulièrement dans les cas familiaux, l'extension évolutive de ces hamartomes a été décrite : il a été suggéré que ces lésions peuvent exprimer des facteurs et/ou des récepteurs impliqués dans l'angiogenèse (Rothbart et al., 1996 ; Notelet et al., 1997).

Il convient d'indiquer que les inventeurs ont observé dans quatre familles (Labauge et al., 1999) que des malformations cutanées (également appelées angiomes) pouvaient ségréger avec les cavernomes cérébraux.

Toutes les mutations rapportées ici conduiraient, si elles étaient traduites, à des protéines Krit1 tronquées qui seraient délétées de la région interagissant avec Rap1A / Krev1.

Les fonctions exactes de Rap1A / Krev1 ne sont pas tout à fait élucidées. Ce membre de la famille des Ras GTPase est exprimé de façon ubiquitaire particulièrement dans les neutrophiles, les plaquettes et le cerveau ; il est localisé dans les compartiments endocytiques / lysosomiaux. Rap1A a été décrit comme interagissant avec B-Raf, ce qui est tout à fait intéressant au vu de l'apoptose massive endothéliale observée chez les souris déficientes pour B-Raf (Wojnowski et al., 1997). *In vivo*, des études sur les eucaryotes inférieurs tels que les levures et la drosophile ont récemment donné quelques indications sur les fonctions de Rap1A dans la différentiation et la morphogenèse (Asha et al.).

L'interaction de Krit1 et de Rap1A suggère que Krit1 pourrait soit réguler Rap1A soit être un effecteur de Rap1A (Bos et al.). Les mutations rapportées ici pourraient résulter soit d'un effet dominant négatif soit d'une perte de fonction. L'observation des familles présentant des délétions complètes de Krit1 serait un argument fort en faveur de cette hypothèse. Par ailleurs le fait que les formes sporadiques de cavernomes se manifestent principalement par une lésion unique et que les formes familiales se manifestent pas des lésions multiples suggère fortement que ces lésions suivent la règle du « *double hit de Knudson* » (Knudson 1971) et qu'une perte complète de fonction de Krit1 pourrait être nécessaire à l'apparition des cavernomes.

En d'autres termes, dans les formes dominantes de la maladie, une première mutation, présente dans toutes les cellules de l'organisme à l'état hétérozygote, serait présente. L'apparition des lésions cavernomateuses serait conditionnée par la survenue d'une deuxième mutation touchant l'autre allèle de ce gène, mutation qui surviendrait de façon somatique. Dans les formes sporadiques les plus étudiées à ce jour, l'individu ne présente aucune mutation germinale et la lésion unique résulterait de deux mutations survenues dans la même cellule.

Cependant, il existerait des formes sporadiques de cavernomes différentes de celles ci-dessus décrites. Les Inventeurs ont en effet mis en évidence une forme sporadique se manifestant par des lésions multiples et résultant d'une mutation *de novo* dans le gène *Krit1* vraisemblablement dans une cellule germinale d'un des deux parents du patient atteint (données non montrées).

En résumé, les données reportées ici, suggèrent fortement que les mutations tronquantes de Krit1 sont responsables de l'apparition des cavernomes cérébraux observés dans les familles *CCM1* mais également dans certaines formes sporadiques, mettant en exergue le rôle putatif de la voie de signalisation de Rap1A dans ces mécanismes.

Parmi les applications thérapeutiques ici décrites, pourraient être concernées différents types de malformations vasculaires, dysplasies vasculaires, angiomes et/ou toute situation où existe une angiogenèse anormale.

Ainsi, l'utilisation du gène *Krit1* ou d'une séquence dérivée de ce gène pour la préparation d'un médicament ou son utilisation dans une approche de type thérapie génique destiné à contrôler ou inhiber l'angiogenèse notamment par surexpression *in situ* du gène *Krit1* ou d'une séquence dérivée de ce gène est décrites ici.

Par « séquence dérivée de ce gène », on entend toute séquence ou partie de séquence normale ou mutée du gène *Krit1* et présentant une activité similaire et comparable à la séquence totale fonctionnelle de référence.

Un vecteur d'expression dans une cellule hôte appropriée comportant la séquence du gène *krit1* ou une séquence dérivée de ce gène (la séquence dérivée est définie ci-dessus) est décrit ici. Dans le cas où l'on souhaiterait réprimer une angiogenèse anormale, il pourrait être intéressant de surexprimer la séquence en question et c'est la raison pour laquelle le vecteur comprend, avantageusement, les éléments nécessaires à cette surexpression.

En particulier, le vecteur peut être destiné à la thérapie génique et dans le cas où l'on souhaiterait limiter son site d'action, ce vecteur peut comporter une séquence assurant le ciblage et/ou l'expression tissu spécifique des séquences qu'il comprend.

Enfin, une composition thérapeutique est décrite comportant à titre de principe actif tout ou partie de la protéine Krit1 normale ou modifiée, de façon à se substituer par exemple à une protéine tronquée et pallier la déficience. Le principe actif peut également être un vecteur tel que décrit précédemment.

La **Figure 1** représente la carte génétique, physique et transcriptionnelle du locus *CCM1.*

La Figure 1a représente la carte génétique du locus *CCM1.* Ce locus était précédemment défini par l'intervalle D7S2410-D7S689. Les intervalles génétiques réduits MS2456-D7S689 sont indiqués par des crochets horizontaux. Les microsatellites déjà publiés sont encadrés. Les nouveaux microsatellites sont identifiés par des caractères gras. Certains des STS sont également montrés. Le STS sWSS 1703 correspond aux nucléotides 393-658 de *Krit1.* Les marqueurs entre les crochets verticaux sont espacés de moins de 1 kb.

La Figure 1b représente la carte physique et transcriptionnelle du locus *CCM1.* Des contigs de BAC sont répartis sur l'intervalle *CCM1.* Le BAC AC000120 est représenté par le trait le plus épais. Les chevauchements avec soit les STS, soit les marqueurs microsatellites sont indiqués par des petites barres verticales. La flèche noire correspond à *Krit1,* les flèches blanches correspondent à des gènes humains bien caractérisés et les flèches vides correspondent à des gènes présentant de fortes homologies avec des gènes d'autres espèces (non caractérisés chez les humains).

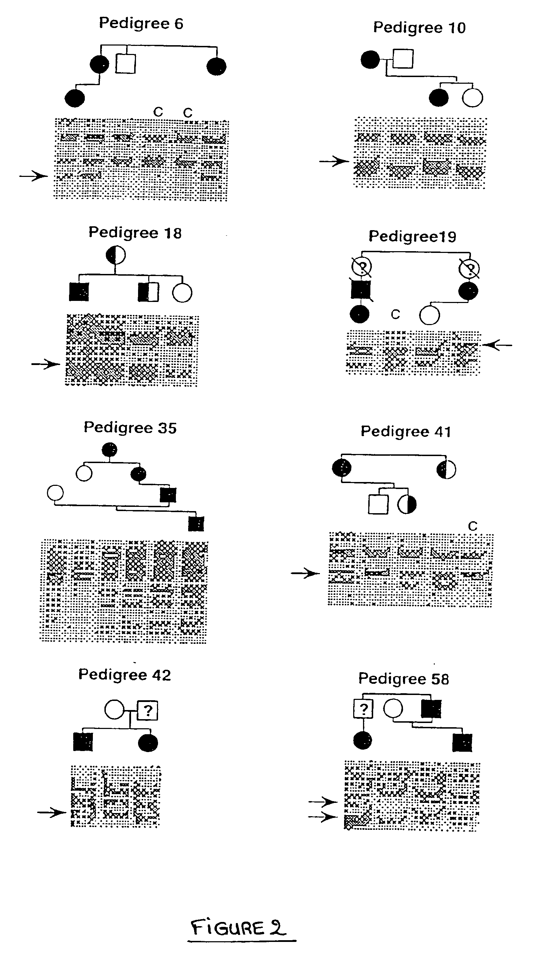

La **Figure 2** représente la coségrégation des variants de conformation avec le phénotype malade au sein de 8 pedigrees (SSCP).

Les symboles vides désignent les sujets dont l'examen du cerveau par IRM est normal, les symboles noirs à moitié pleins correspondent à des patients asymptomatiques présentant des cavernomes sur l'examen par IRM, les symboles noirs pleins correspondent à des patients symptomatiques et présentant des cavernomes sur l'IRM ; le signe « ? » correspond aux sujets ayant un statut inconnu et le signe « \ » correspond à des patients décédés. Les patients décédés ou avec un statut inconnu n'ont pas été testés pour la mutation mais sont représentés pour une meilleure compréhension des structures familiales. Les bandes anormales sont indiquées par une flèche.

La **Figure 3** illustre les mutations de *Krit1.*

La Figure 3a représente la structure du gène *Krit1* et de la protéine putative correspondante. « ns » signifie non-sens, «del» signifie délétion et « ins » signifie insertion. Pour les insertions, le numéro de nucléotide correspond au nucléotide précédant l'insertion. L'expression « Krev Interacting Region » correspond à la région (acides aminés 483-529) dont la délétion abolit l'interaction avec Krev lors du test en double hybride dans la levure.

La Figure 3b représente les mutations de *Krit1,* identifiées dans les 8 pedigrees mentionnés plus haut. Les flèches indiquent les sites de mutation. WT signifie séquence de type sauvage et MT signifie séquence mutante. Dans le pedigree 42, le chromatogramme et la séquence présentés correspondent au brin négatif; le brin positif a montré une superposition complète des séquences normales et anormales et ne permettaient pas d'avoir une bonne visualisation du site du début de la délétion.

### EXEMPLES

### MATERIELS ET METHODES

### Patients

20 patients non apparentés appartenant à des familles dont on savait qu'elles présentaient de façon héréditaire des cavernomes ont fait partie de l'étude avec leur libre consentement (Labauge et al.).

L'analyse de ce panel de familles avec le test HOMOG a montré que ces familles avaient une probabilité *a posteriori* d'être liées au locus *CCM1* supérieure à 90 % (Laberge et al.).

Une approche combinant biologie moléculaire et bio informatique a été utilisée pour établir la carte physique et transcriptionnelle du locus *CCM1.* Après validation d'un contig de YACs préalablement publiée par Johnson et al. (1995), les auteurs ont positionné dans l'intervalle par PCR et par une approche *in silico* 574 EST (Expressed Sequence Tags) qu'ils ont regroupés en 53 groupes.

### Réduction de l'intervalle génétique

12 marqueurs microsatellites polymorphes recouvrant l'intervalle D7S2410-D7S689 ont été sélectionnés pour les analyses de liaison. 7 d'entre eux étaient préalablement connus : D7S2410, D7S2409, D7S1813, D7S1789, D7S646, D7S689 et D7S558, et ont été utilisés par plusieurs équipes (Günel et al. dans Neurosurgery*,* Craig et al., Labauge et al., Laberge et al.). Les derniers MS65, MS2453A, MS2456A, MS119 et MS120 ont été identifiés par les inventeurs sur la base des données de séquence des BAC cartographiés dans l'intervalle.

### Détection des mutations et identification

Sur la base de la comparaison des séquences de l'ADNc de Krit1 et du BAC AC 00000120, les inventeurs ont déterminé 14 jeux d'amorces pour amplifier les 12 exons et les sites de jonction exon / intron de *Krit1* à partir de l'ADN génomique. Des réactions de PCR ont été mises en oeuvre comme suit : après une première étape initiale de dénaturation à 94°C (4 min), 30 cycles d'amplification consistant en des étapes à 94°C (15 s), une température d'hybridation optimisée comprise entre 45°C et 55°C (15s) et 72°C (15s) suivie par une étape d'extension finale à 72°C (10 min). Les produits de PCR ont été soumis à une électrophorèse dans 4 types de conditions (acrylamide à 10 % avec ou sans glycérol à 4°C et 20°C) sur un appareil Mighty Small II (Pharmacia-Biogen) utilisé en condition de courant constant de 35 mA. Des variants de conformation ont été révélés à l'argent (Silver Stain Plus kit, Biorad). Des amplimères montrant un pattern de bandes SSCP atypiques ont été séquencés (AB1377, Perkin Elmer). Toutes les mutations décelées lors du séquençage ont été testées pour leur coségrégation avec le phénotype malade par une approche SSCP.

**TABLEAU 1 : AMORCES**

| **EXON** | **AMORCE SENS** | **AMORCE REVERSE** | **TAILLE DE L'AMPLI-MERE** |
|---|---|---|---|
| 1 | GAGCGGATAAAAACTAAT (SEQ ID N° 1) | GAGCTAAAATTCATTCAA (SEQ ID N° 2) | 205 |
| 2 | GCTCTTAATGGGTTTTTG (SEQ ID N° 3) | AGCAATGTGGAGTAAAAC (SEQ ID N° 4) | 183 |
| 3 | TTTGGAATGAGAACAGTC (SEQ ID N° 5) | GTCCTGTTGTATTTTTCA (SEQ ID N° 6) | 265 |
| 4 | GTTGTTGTTTTTTGTTTG (SEQ ID N °7) | ACCTGGAAAATAACTTAC (SEQ ID N° 8) | 208 |
| 5 | ATGTAATGCCTTTTTTCC (SEQ ID N° 9) | ATGCCTGGCTCTAACTAT (SEQ ID N° 10) | 181 |
| 6 | TTGTTAGATTGTGATGTA (SEQ ID N° 11) | AACATAATAAAAACTTTC (SEQ ID N° 12) | 257 |
| 7 | TTTATAAAAGGAATGATG (SEQ ID N° 13) | TCAACTCAAACCATATCA (SEQ ID N° 14) | 335 |
| 8 | TGTAGCCTAATAACCAAA (SEQ ID N° 15) | AGCATAGCACAAGACCAT (SEQ ID N° 16) | 243 |
| 9 | GGTGAAGTTTTTAATATG (SEQ ID N° 17) | CAATAGTTTATGAAGTCC (SEQ ID N° 18) | 213 |
| 10 | ATATTTACAAAGGCAAGC (SEQ ID N° 19) | TGACATGATTGGTAAAAA (SEQ ID N° 20) | 180 |
| | TGGTACATTTTCCTTTCA (SEQ ID N° 21) | CTTTATGATTGCTGGGGC (SEQ ID N° 22) | 201 |
| 11 | GGTGAAGTTTTTAATATG (SEQ ID N° 23) | CAATAGTTTATGAAGTCC (SEQ ID N° 24) | 205 |
| 12 | AATAGATAGGGAACTGCC (SEQ ID N° 25) | GTGGCTTGAGTAACAGTT (SEQ ID N° 26) | 234 |
| | TAATGCCCACTGAAAGAA (SEQ ID N° 27) | GGCTGGTCTTGAACTCTG (SEQ ID N° 28) | 199 |

**TABLEAU 2 : SEQUENCES DES JONCTIONS INTRON-EXON**

| **EXON** | **POSITION SUR L'ADNc** | **TAILLE** | **SEQUENCE** | | **POSITION SUR BAC AC000120** |
|---|---|---|---|---|---|
| 1 | 8-133 | 126 | atcaggtcag ACAGAAAACT... (SEQ ID N° 29) | TACAAATCGG gtaagagttg (SEQ ID N° 30) | 127165-127040 |
| 2 | 134-249 | 116 | ccctttctag GTAGATAAAG... (SEQ ID N° 31) | CAGAAGACAA gtactgtttc (SEQ ID N° 32) | 126561-126445 |
| 3 | 250-393 | 144 | taatgattag GGAACGACAG... (SEQ ID N° 33) | ATGCATGCTG gtaaatggaa (SEQ ID N° 34) | 126228-126086 |
| 4 | 394-550 | 157 | ttttatacag GTATGGAAAA... (SEQ ID N° 35) | AACGGATAGA gtaagttatt (SEQ ID N° 36) | 118319-118163 |
| 5 | 551-657 | 107 | acatttctag CATATAACAG... (SEQ ID N° 37) | TAACAAACCA gtaagaatta (SEQ ID N° 38) | 117464-117357 |
| 6 | 658-815 | 157 | tttcttgtag TATGAAAAAG... (SEQ ID N° 39) | GAAAACCTCA gtaagaaagt (SEQ ID N° 40) | 114615-114459 |
| 7 | 816-967 | 152 | tgtttttcag GCCTTCAACT... (SEQ ID N° 41) | TGAAAAACAG gtttgcttgg (SEQ ID N° 42) | 113690-113539 |
| 8 | 968-1134 | 168 | ttcctttaag ATTGAAGACC... (SEQ ID N° 43) | GTTTCCTAAA gtaagtattt (SEQ ID N° 44) | 106414-106248 |
| 9 | 1135-1222 | 88 | gtgcttacag TGAAGAAAAT... (SEQ ID N° 45) | TGAATACAAG gtaagctgtt (SEQ ID N° 46) | 105616-105529 |
| 10 | 1223-1429 | 207 | ttgtttttag AATCTCAGTA... (SEQ IDN ° 47) | GGAAACTAAG gtagattttc (SEQ ID N° 48) | 105038-104832 |
| 11 | 1430-1546 | 117 | tatgttgcag GCTTTACTCA... (SEQ ID N° 49) | TACAAAACAG gtaagtatca (SEQ ID N° 50) | 93060-92942 |
| 12 | 1547-2004^{*} | 458* | tactttgtag GCTCTGGTCG...* (SEQ IDN° 51) | | 92441-91984^{*} |

| | | | | | |
|---|---|---|---|---|---|
| * Exon 12 non entièrement déterminé car contient des séquences *Alu* | | | | | |

**TABLEAU 3 : MUTATIONS DANS KRIT1**

| **PEDIGREE** | **MUTATIONS DANS L'ADN GENOMIQUE** | **MUTATIONS DES ACIDES AMINES** | |
|---|---|---|---|
| 6 | Délétion (A) | Changements en acides aminés après AA 438 | Normal (WT): IF (438) TKASPSNHKVIPVIVG... |
| | nt 1342 | | Mutant (MT): IF (438) TIRTAPAIIKSSLM*stop codon |
| | Exon 10 | | |
| 10 | Faux-sens (C → T) | Changements en acides aminés après AA 420 | WT : FL (420) QN... |
| | nt 1283 | | MT : FL (420) * stop codon |
| | Exon 10 | | |
| 18 | Insertion (C) | Changements en acides aminés après AA 74 | WT : ED (74) KERQWDD... |
| | après nt 247 | | MT : ED (74) QGTTVGR*stop codon |
| | Exon 2 | | |
| 19 | Faux-sens (G → A) | Changements en acides aminés après AA 79 | WT : RQ (79) WVDD... |
| | nt 261 | | MT : RQ (79) * stop codon |
| | Exon 3 | | |
| 35 | Délétion (26 pb) | Changements en acides aminés après AA 328 | WT : EA (328) RYNLLKGFYTAPDAKL... |
| | nt 1012 | | MT : EA (328) SSS* stop codon |
| | Exon 8 | | |
| 41 | Faux-sens (G → A) | Changements en acides aminés après AA 197 | WT : NN (197) WEEAA... |
| | nt 615 | | MT : NN (197) * stop codon |
| | Exon 5 | | |
| 42 | Délétion (GAAT) | Changements en acides aminés après AA 217 | WT : IY (217) RMDGSYRSVELK... |
| | nt 681-684 | | MT : IY (217) RMGHIVLLN*stop codon |
| | Exon 6 | | |
| 58 | Insertion (C) | Changements en acides aminés après AA 415 | WT : LQ (415) RMFLQNCQIFTKASPSNHKV... |
| | après nt 1271 | | MT : LQ(415) HVLTQLTDIKGYKPQQS*stop codon |
| | Exon 10 | | |

### REFERENCES

1. Altschul, S.F., Madden T.L., Schaffer A. A. et al. Gapped BLAST and PSI-BLAST : a new generation of protein database search programs. Nucleic Acids Res. 25 : 3389-3402. (1997)
2. Asha H., de Ruiter N.D., Wang M.G. and Hariharan I.K. The Rap1 GTPase functions as a regulator of morphogenesis in vivo. EMBO, 18 : 605-615 (1999)
3. Bos J.L. All in the familly ? New insights and questions regarding interconnectivity of Ras, Rap1 and Ral. EMBO, 17 :6776-6782 (1998)
4. Craig H.D., Günel M., Cepada O. et al. Multilocus linkage identifies two new loci for a Mendelian form of stroke, cerebral cavernous malformation, at 7p1(-13 and 3q25.2-27. Hum Mol Genet 7: 1851-1858. (1998)
5. Dubovsky J., Zabramsky J.M., Kurth J. et al. A gene responsible for cavenous malformations of the brain maps to chromosome 7q. Hum Mol Genet 4 : 453-458. (1995)
6. Ducros A., Denier C., Joutel A. et al. Recurrence of the T666M Calcium Channel CACNAIA Gene Mutation in Familial Hemiplegic Migraine with Progressive Cerebellar Ataxia. Am JHum Genet, 64 : 89-98. (1999)
7. Eenson DA, Boguski MS, Lipman DJ et al. GenBank. Nucleic Acids Res 27 :12-7 (1999)
8. Günel M., Awad I.A., Finberg K.S. et al. A Founder Mutation as a cause of cerebral cavernous malformation in Hispanic Americans. N Eng J Med 334 : 946-951. (1996)
9. Günel M, Awad IA, Finberg K et al. Genetic heterogeneity of inherited cerebral cavernous malformation. Neurosurgery 38 : 1265-1271. (1996)
10. Henkemeyer M., Rossi D.J., Holmyard D.P. et al. Vascular system defects and neuronal apoptosis in mice lacking ras GTPase-activing protein. Nature 377 : 695-701. (1995)
11. Huang X., Adams M.D., Zhou H. and Kerlavage A.R. A tool for analysing and annotating genomic sequences Genomics 46 : 37-45 (1997)
12. Johnson EW, Iyer LM, Rich SS et al. Refined Localization of the Cerebral Cavemous Malformation Gene (CCM1) to a 4 cM Interval of Chromosome 7q Contained in a Well-defined YAC Contig. Genome Research, 5 : 368-380. (1995)
13. Kiayama H., Sugimoto Y., Matsuzaki T., Ikawa Y and Noda M. A ras-Related gene with Transformation Suppressor Activity. Cell, 56 : 77-84 (1989)
14. Knudson AG Mutation and Cancer : Statistical study of retinoblastoma ; Proc. Nat. Ac. Sci. USA 68, 820-823 (1971).
15. Labauge P., Laberge S., Brunereau L. et al. Hereditary cerebral cavernous angiomas : clinical and genetic features in 57 French familles. Lancet 352 : 1892-258. (1998)
16. Laberge S., Labauge P., Maréchal E., Maciazek J, Tournier-Lasserve E. Genetic heterogeneity and absence of founder effect in a series of 36 non Hispano-American cerebral cavernomas families. (European Journal of Human Genetics, in press)
17. Notelet L., Houtteville J.P., Khoury S., Lechevalier B. and Chapon F. Proliferating cell nuclear antigen (PCNA) in cerebral cavenomas : ans immunocytochemical study of 42 cases. Surg Neurol 47 : 364-70 (1997)
18. Oten P, Pizzolata GP, Rilliet B, Berney J. A propos de 131 cas d'angiomes caverneux (cavernomes) du SNC, repérés par l'analyse rétrospective de 24 535 autopsies. Neurochirurgie 35 :82-83 (1989)
19. Pizon V., Chardin P., Lerosey I., Olofsson B. and Tavitian A. Human cDNAs rap1 and rap2 homologous to the Drosophilia gene Dras3 encode proteins closely related to ras in the effector region. Oncogene, 3 : 201-204 (1988)
20. Pizon V., Cifuentes-Diaz C., Mege RM., Baldacci G. and Rieger F. Expression and localization of Rap1 proteins during myogenic differentiation. Eur J Cell Biol, 69 : 224-235 (1996)
21. Quarck R., Berrou E., Magnier C., Bobe R., Bredoux R., Tobelem G., Enouf Eand Bryckaert M. Differential up-regulation of Rapla and Raplb proteins during smooth muscle cell cycle. Eur J Cell Biol 70 : 269-277 (1996)
22. Rigamonti D, Hadley MN, Drayer BP et al. Cerebral cavernous malformations. Incidence and familial occurrence. N Engl J Med 319 : 343-347. (1988)
23. Risau W. Mechanisms of angiogenesis. Nature 386 : 671-674. (1997)
24. Russell DS, Rubenstein LJ. Pathology of tumours of the nervous system. 5th ed. Baltimore, Md ; Williams and Wilkins :730-736. (1989)
25. Rothbart D., Awad I.A., Lee J., Kim J., Harbaugh R., Crisculo G.R. Expression of Angiogenic Factors and Structural Proteins in Central Nervous System Vascular Malformations. Neurosurgery 38 : 915-925 (1996)
26. Rozen S., Skaletsky H.J. Primer 3 http://wwwgenome.wi.mit.edu/genome_software/other/primer3.html (1996, 1997)
27. SerebriiskiiI., Estojak J., Sonoda G. et al. Association of Krev1/rap1a with Krit1, a novel ankyrin repeat-containing protein encoded by a gene mapping to 7q21-22 Oncongene 15 : 1043-1049. (1997)
28. Wojnowski L., Zimmer A.M., Beck T.W. et al. Endothelial apoptosis in Braf-deficient mice. Nature Genet 16 : 293-297. (1997)
29. Xu HP., Wang Y., Riggs M., Rodgers L. and Wigler M. Biological activity of the mammalian RAP genes in yeast. Cell Regul 1 :763-9 (1990)

## Revendications

1. Méthode de diagnostic génotypique de cavernomes chez un individu, **caractérisée par le fait que** l'on prélève un échantillon biologique dudit individu, que l'on détecte la présence d'une mutation dans le gène *Krit1* conduisant à l'expression d'une protéine Krit1 tronquée par analyse de la séquence nucléique présente dans ledit échantillon, une telle mutation étant liée à la survenue de cavernomes.

2. Méthode de diagnostic selon la revendication 1, **caractérisée par le fait que** la séquence d'acide nucléique est de l'ADN génomique, de l'ADNc ou de l'ARNm.

3. Méthode de diagnostic selon l'une des revendications 1 ou 2, **caractérisée par le fait que** ladite analyse est réalisée par hybridation.

4. Méthode de diagnostic selon l'une des revendications 1 à 3, **caractérisée par le fait que** ladite analyse est réalisée par séquençage.

5. Méthode de diagnostic selon l'une des revendications 1 ou 2, **caractérisée par le fait que** ladite analyse est réalisée par migration électrophorétique, et plus particulièrement par SSCP ou DGGE.

6. Méthode de diagnostic selon l'une des revendications 1 à 5, **caractérisée par le fait que** tout ou partie de la séquence d'acide nucléique correspondant au gène *Krit1* est amplifiée préalablement à la détection de la présence d'une mutation.

7. Méthode de diagnostic selon la revendication 6, **caractérisée par le fait que** l'amplification est réalisée par PCR.

8. Méthode de diagnostic selon la revendication 7, **caractérisée par le fait que** les amorces pour réaliser l'amplification sont parmi les séquences choisies dans le groupe comprenant SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEQ ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28.

9. Méthode de diagnostic selon les revendications 7 ou 8, **caractérisée par le fait que** les couples d'amorces pour réaliser l'amplification sont choisis parmi le groupe comprenant :
- SEQ ID N° 1 / SEQ ID N° 2 pour l'exon 1,
- SEQ ID N° 3 / SEQ ID N° 4 pour l'exon 2,
- SEQ ID N° 5 / SEQ ID N° 6 pour l'exon 3,
- SEQ ID N° 7 / SEQ ID N° 8 pour l'exon 4,
- SEQ ID N° 9 / SEQ ID N° 10 pour l'exon 5,
- SEQ ID N° 11 / SEQ ID N° 12 pour l'exon 6,
- SEQ ID N° 13 / SEQ ID N° 14 pour l'exon 7,
- SEQ ID N° 15 / SEQ ID N° 16 pour l'exon 8,
- SEQ ID N° 17 / SEQ ID N° 18 pour l'exon 9,
- SEQ ID N° 19 / SEQ ID N° 20 pour l'exon 10,
- SEQ ID N° 21 / SEQ ID N° 22 pour l'exon 10,
- SEQ ID N° 23 / SEQ ID N° 24 pour l'exon 11,
- SEQ ID N° 25 / SEQ ID N° 26 pour l'exon 12,
- SEQ ID N° 27 / SEQ ID N° 28 pour l'exon 12.

10. Utilisation d'au moins une amorce de séquence nucléotidique pour la détection, à partir d'un prélèvement biologique, de la présence d'une mutation dans le gène *Krit1* susceptible de conduire à une protéine Krit1 tronquée, et ladite amorce étant choisie dans le groupe comprenant SEQ ID N° 1, SEQ ID N° 2, SEQ ID N° 3, SEQ ID N° 4, SEQ ID N° 5, SEQ ID N° 6, SEQ ID N° 7, SEQ ID N° 8, SEQ ID N° 9, SEQ ID N° 10, SEQ ID N° 11, SEQ ID N° 12, SEQ ID N° 13, SEQ ID N° 14, SEQ ID N° 15, SEQ ID N° 16, SEQ ID N° 17, SEQ ID N° 18, SEUL ID N° 19, SEQ ID N° 20, SEQ ID N° 21, SEQ ID N° 22, SEQ ID N° 23, SEQ ID N° 24, SEQ ID N° 25, SEQ ID N° 26, SEQ ID N° 27, SEQ ID N° 28.

11. Utilisation selon la revendication 10, pour la détection, à partir d'un prélèvement biologique, de la présence d'une mutation dans le gène *Krit1* liée à la survenue de cavernomes.

12. Utilisation selon l'une des revendications 10 et 11, **caractérisée en ce que** la détection de la mutation est effectuée dans au moins un exon du gène *Krit1*.

13. Utilisation selon la revendication 12, **caractérisée par le fait qu'**un couple de séquences est spécifique d'un exon selon la répartition suivante :
- SEQ ID N° 1 / SEQ ID N° 2 pour l'exon 1,
- SEQ ID N° 3 / SEQ ID N° 4 pour l'exon 2,
- SEQ ID N° 5 / SEQ ID N° 6 pour l'exon 3,
- SEQ ID N° 7 / SEQ ID N° 8 pour l'exon 4,
- SEQ ID N° 9 / SEQ ID N° 10 pour l'exon 5,
- SEQ ID N° 11 / SEQ ID N° 12 pour l'exon 6,
- SEQ ID N° 13 / SEQ ID N° 14 pour l'exon 7,
- SEQ ID N° 15 / SEQ ID N° 16 pour l'exon 8,
- SEQ ID N° 17 / SEQ ID N° 18 pour l'exon 9,
- SEQ ID N° 19 / SEQ ID N° 20 pour l'exon 10,
- SEQ ID N° 21 / SEQ ID N° 22 pour l'exon 10,
- SEQ ID N° 23 / SEQ ID N° 24 pour l'exon 11,
- SEQ ID N° 25 / SEQ ID N° 26 pour l'exon 12,
- SEQ ID N° 27 / SEQ ID N° 28 pour l'exon 12.

14. Utilisation selon l'une des revendications 10 à 13, **caractérisée par le fait que** la détection d'une mutation est précédée de l'amplification de l'exon dans lequel la mutation est recherchée.

15. Utilisation selon la revendication 14, **caractérisée par le fait que** l'amplification est réalisée par PCR.

## Patentansprüche

1. Verfahren zur genotypischen Diagnose von Kavernomen bei einer Person, **dadurch gekennzeichnet, dass** eine biologische Probe von der Person genommen wird und das Vorliegen einer Mutation in dem *Krit1-Gen,* die zur Expression eines verkürzten Krit1-Proteins führt, durch Analyse der Nukleinsäuresequenz, die in der Probe vorliegt, nachgewiesen wird, wobei eine derartige Mutation mit dem Auftreten von Kavernomen assoziiert ist.

2. Diagnoseverfahren nach Anspruch 1, **dadurch gekennzeichnet, dass** die Nukleinsäuresequenz genomische DNA, cDNA oder mRNA ist.

3. Diagnoseverfahren nach einem der Ansprüche 1 oder 2, **dadurch gekennzeichnet, dass** die Analyse mittels Hybridisierung durchgeführt wird.

4. Diagnoseverfahren nach einem der Ansprüche 1 bis 3, **dadurch gekennzeichnet, dass** die Analyse mittels Sequenzierung durchgeführt wird.

5. Diagnoseverfahren nach einem der Ansprüche 1 oder 2, **dadurch gekennzeichnet, dass** die Analyse mittels elektrophoretischer Wanderung und insbesondere mittels SSCP oder DGGE durchgeführt wird.

6. Diagnoseverfahren nach einem der Ansprüche 1 bis 5, **dadurch gekennzeichnet, dass** die gesamte oder ein Teil der Nukleinsäuresequenz, die dem *Krit1*-Gen entspricht, vor dem Nachweis des Vorliegens einer Mutation amplifiziert wird.

7. Diagnoseverfahren nach Anspruch 6, **dadurch gekennzeichnet, dass** die Amplifikation mittels PCR durchgeführt wird.

8. Diagnoseverfahren nach Anspruch 7, **dadurch gekennzeichnet, dass** die Primer zum Durchführen der Amplifikation zu den Sequenzen gehören, die aus der Gruppe ausgewählt sind, die SEQ ID Nr. 1, SEQ ID Nr. 2, SEQ ID Nr. 3, SEQ ID Nr. 4, SEQ ID Nr. 5, SEQ ID Nr. 6, SEQ ID Nr. 7, SEQ ID Nr. 8, SEQ ID Nr. 9, SEQ ID Nr. 10, SEQ ID Nr. 11, SEQ ID Nr. 12, SEQ ID Nr. 13, SEQ ID Nr. 14, SEQ ID Nr. 15, SEQ ID Nr. 16, SEQ ID Nr. 17, SEQ ID Nr. 18, SEQ ID Nr. 19, SEQ ID Nr. 20, SEQ ID Nr. 21, SEQ ID Nr. 22, SEQ ID Nr. 23, SEQ ID Nr. 24, SEQ ID Nr. 25, SEQ ID Nr. 26, SEQ ID Nr. 27 und SEQ ID Nr. 28 umfasst.

9. Diagnoseverfahren nach Anspruch 7 oder 8, **dadurch gekennzeichnet, dass** die Primerpaare zum Durchführen der Amplifikation aus der Gruppe ausgewählt sind, die folgende umfasst:
- SEQ ID Nr. 1 / SEQ ID Nr. 2 für das Exon 1,
- SEQ ID Nr. 3 / SEQ ID Nr. 4 für das Exon 2,
- SEQ ID Nr. 5 / SEQ ID Nr. 6 für das Exon 3,
- SEQ ID Nr. 7 / SEQ ID Nr. 8 für das Exon 4,
- SEQ ID Nr. 9 / SEQ ID Nr. 10 für das Exon 5,
- SEQ ID Nr. 11 / SEQ ID Nr. 12 für das Exon. 6,
- SEQ ID Nr. 13 / SEQ ID Nr. 14 für das Exon 7,
- SEQ ID Nr. 15 / SEQ ID Nr. 16 für das Exon 8,
- SEQ ID Nr. 17 / SEQ ID Nr. 18 für das Exon 9,
- SEQ ID Nr. 19 / SEQ ID Nr. 20 für das Exon 10,
- SEQ ID Nr. 21 / SEQ ID Nr. 22 für das Exon 10,
- SEQ ID Nr. 23 / SEQ ID Nr. 24 für das Exon 11,
- SEQ ID Nr. 25 / SEQ ID Nr. 26 für das Exon 12,
- SEQ ID Nr. 27 / SEQ ID Nr. 28 für das Exon 12.

10. Verwendung mindestens eines Nukleotidsequenz-Primers zum Nachweis des Vorliegens einer Mutation in dem *Krit1-*Gen, die zu einem verkürzten Krit1-Protein führen kann, ausgehend von einer biologischen Probe, wobei der Primer aus der Gruppe ausgewählt ist, die SEQ ID Nr. 1, SEQ ID Nr. 2, SEQ ID Nr. 3, SEQ ID Nr. 4, SEQ ID Nr. 5, SEQ ID Nr. 6, SEQ ID Nr. 7, SEQ ID Nr. 8, SEQ ID Nr. 9, SEQ ID Nr. 10, SEQ ID Nr. 11, SEQ ID Nr. 12, SEQ ID Nr. 13, SEQ ID Nr. 14, SEQ ID Nr. 15, SEQ ID Nr. 16, SEQ ID Nr. 17, SEQ ID Nr. 18, SEQ ID Nr. 19, SEQ ID Nr. 20, SEQ ID Nr. 21, SEQ ID Nr. 22, SEQ ID Nr. 23, SEQ ID Nr. 24, SEQ ID Nr. 25, SEQ ID Nr. 26, SEQ ID Nr. 27 und SEQ ID Nr. 28 umfasst.

11. Verwendung nach Anspruch 10 zum Nachweis des Vorliegens einer Mutation in dem Kritl-Gen, die mit dem Auftreten von Kavernomen assoziiert ist, ausgehend von einer biologischen Probe.

12. Verwendung nach einem der Ansprüche 10 und 11, **dadurch gekennzeichnet, dass** der Nachweis der Mutation in mindestens einem Exon des *Krit1*-Gens vorgenommen wird.

13. Verwendung nach Anspruch 12, **dadurch gekennzeichnet, dass** ein Paar von Sequenzen gemäß der folgenden Aufteilung für ein Exon spezifisch ist:
- SEQ ID Nr. 1 / SEQ ID Nr. 2 für das Exon 1,
- SEQ ID Nr. 3 / SEQ ID Nr. 4 für das Exon 2,
- SEQ ID Nr. 5 / SEQ ID Nr. 6 für das Exon 3,
- SEQ ID Nr. 7 / SEQ ID Nr. 8 für das Exon 4,
- SEQ ID Nr. 9 / SEQ ID Nr. 10 für das Exon 5,
- SEQ ID Nr. 11 / SEQ ID Nr. 12 für das Exon 6,
- SEQ ID Nr. 13 / SEQ ID Nr. 14 für das Exon 7,
- SEQ ID Nr. 15 / SEQ ID Nr. 16 für das Exon 8,
- SEQ ID Nr. 17 / SEQ ID Nr. 18 für das Exon 9,
- SEQ ID Nr. 19 / SEQ ID Nr. 20 für das Exon 10,
- SEQ ID Nr. 21 / SEQ ID Nr. 22 für das Exon 10,
- SEQ ID Nr. 23 / SEQ ID Nr. 24 für das Exon 11,
- SEQ ID Nr. 25 / SEQ ID Nr. 26 für das Exon 12,
- SEQ ID Nr. 27 / SEQ ID Nr. 28 für das Exon 12.

14. Verwendung nach einem der Ansprüche 10 bis 13, **dadurch gekennzeichnet, dass** dem Nachweis einer Mutation die Amplifikation des Exons, in der die Mutation gesucht wird, vorausgeht.

15. Verwendung nach Anspruch 14, **dadurch gekennzeichnet, dass** die Amplifikation mittels PCR durchgeführt wird.

## Claims

1. A method for genotypically diagnosing cavernomas in an individual, **characterized in that** a biological sample is taken from said individual, and **in that** the presence of a mutation in the *Krit1* gene leading to the Expression of a truncated *Krit1* protein is detected by analyzing the nucleic acid sequence present in said sample, such a mutation being linked to the occurrence of cavernomas.

2. The diagnostic method according to claim 1, **characterized in that** the nucleic acid sequence is genomic DNA, cDNA or mRNA.

3. The diagnostic method according to one of claims 1 and 2, **characterized in that** said analysis is carried out by hybridization.

4. The diagnostic method according to one of claims 1 to 3, **characterized in that** said analysis is carried out by sequencing.

5. The diagnostic method according to one of claims 1 and 2, **characterized in that** said analysis is carried out by electrophoretic migration, and more particularly by SSCP or DGGE.

6. The diagnostic method according to one of claims 1 to 5, **characterized in that** all or part of the nucleic acid sequence corresponding to the *Krit1* gene is amplified prior to detecting the presence of a mutation.

7. The diagnostic method according to claim 6, **characterized in that** the amplification is carried out by PCR.

8. The diagnostic method according to claim 7, **characterized in that** the primers for carrying out the amplification are from the sequences chosen from the group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27 and SEQ ID No. 28.

9. The diagnostic method according to claims 7 or 8, **characterized in that** the primer pairs for carrying out the amplification are chosen from the group comprising:
- SEQ ID No. 1/SEQ ID No. 2 for exon 1,
- SEQ ID No. 3/SEQ ID No. 4 for exon 2,
- SEQ ID No. 5/SEQ ID No. 6 for exon 3,
- SEQ ID No. 7/SEQ ID No. 8 for exon 4,
- SEQ ID No. 9/SEQ ID No. 10 for exon 5,
- SEQ ID No. 11/SEQ ID No. 12 for exon 6,
- SEQ ID No. 13/SEQ ID No. 14 for exon 7,
- SEQ ID No. 15/SEQ ID No. 16 for exon 8,
- SEQ ID No. 17/SEQ ID No. 18 for exon 9,
- SEQ ID No. 19/SEQ ID No. 20 for exon 10,
- SEQ ID No. 21/SEQ ID No. 22 for exon 10,
- SEQ ID No. 23/SEQ ID No. 24 for exon 11,
- SEQ ID No. 25/SEQ ID No. 26 for exon 12,
- SEQ ID No. 27/SEQ ID No. 28 for exon 12.

10. Use of at least one nucleotide sequence primer for detecting, from a biological sample, the presence of a mutation in the *Krit1* gene likely to lead to a truncated *Krit1* protein, said primer being chosen from the group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 1.4, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27 and SEQ ID No. 28.

11. The use according to claim 10, for detecting, from a biological sample, the presence of a mutation in the *Krit1* gene linked to the occurrence of cavernomas.

12. The use according to one of claims 10 and 11, **characterized in that** the detection of the mutation takes place in at least one exon of the *Krit1* gene.

13. The use according to claim 12, **characterized in that** a pair of sequences is specific for an exon according to the following distribution:
- SEQ ID No. 1/SEQ ID No. 2 for exon 1,
- SEQ ID No. 3/SEQ ID No. 4 for exon 2,
- SEQ ID No. 5/SEQ ID No. 6 for exon 3,
- SEQ ID No. 7/SEQ ID No. 8 for exon 4,
- SEQ ID No. 9/SEQ ID No. 10 for exon 5,
- SEQ ID No. 11/SEQ ID No. 12 for exon 6,
- SEQ ID No. 13/SEQ ID No. 14 for exon 7,
- SEQ ID No. 15/SEQ ID No. 16 for exon 8,
- SEQ ID No. 17/SEQ ID No. 18 for exon 9,
- SEQ ID No. 19/SEQ ID No. 20 for exon 10,
- SEQ ID No. 21/SEQ ID No. 22 for exon 10,
- SEQ ID No. 23/SEQ ID No. 24 for exon 11,
- SEQ ID No. 25/SEQ ID No. 26 for exon 12,
- SEQ ID No. 27/SEQ ID No. 28 for exon 12.

14. The use according to one of claims 10 to 13, **characterized in that** the detection of a mutation is preceded by amplification of the exon in which the mutation is being sought.

15. The use according to claim 14, **characterized in that** the amplification is carried out by PCR.
